# EUROPEAN PATENT APPLICATION

(11) **EP 4 751 663 A1**
(43) Date of publication of application: **03.06.2026**
(21) Application number: 25219959.1
(22) Date of filing: 02.12.2025
(51) Int. Cl.: A61B 17/072

(54) **SURGICAL STAPLING INSTRUMENT AND ATTACHMENT AND DETACHMENT METHOD THEREOF**

(30) Priority: 02.12.2024 CN 202411746116
(71) Applicant: Ezisurg Medical Co., Ltd., Shanghai 201315 (CN); Ezisurg (Suzhou) Medical Co., Ltd., Suzhou, Jiangsu 215163 (CN)
(72) Inventor: NIE, Honglin, Shanghai, 201315 (CN); YANG, Guang, Shanghai, 201315 (CN); TANG, Chuangang, Shanghai, 201315 (CN)
(74) Representative: EIP

(57) **Abstract**

The present disclosure provides a surgical stapling instrument (10) and an attachment and detachment method thereof. The surgical stapling instrument (10) includes a fixed anvil assembly (32), a cartridge assembly (31) and an end component (40). The attachment and detachment method of the surgical stapling instrument (10) includes: causing the distal portion (324) to press the elastic member (43) to deform; causing the distal portion (324) to be fully inserted into the lumen (41) and to engage with the restored elastic member (43); causing the distal portion (324) to press the elastic member (43) to deform, and to be partially withdrawn from the lumen (41); causing the distal portion (324) to be fully withdrawn from the lumen (41) and to disengage from the restored elastic member (43). The attachment and detachment of the end component (40) of the surgical stapling instrument (10) are relatively simple.

## Description

### TECHNICAL FIELD

The present disclosure relates to a surgical instrument, in particular to a surgical stapling instrument and an attachment and detachment method thereof.

### BACKGROUND

A surgical stapling instrument suitable for surgical procedures is a surgical instrument that is capable of excising excess tissue while suturing a patient's wound, and it is widely applied in minimally invasive procedures in abdominal surgery, gynecology, pediatrics, and thoracic surgery for tissue resection and anastomosis. The surgical stapling instrument is introduced into the body through a cannula of a trocar that is precisely positioned at the surgical site, and subsequently creates a longitudinal incision in the tissue and applies staples to opposite sides of the incision, thereby performing tissue transection and anastomosis similar to a desk stapler.

In general, the surgical stapling instrument includes an actuator, the actuator including a staple cartridge assembly and an anvil assembly. The staple cartridge assembly is used for mounting staples, with the staples oriented toward the anvil assembly, and the staple cartridge assembly is capable of rotating relative to the anvil assembly so as to clamp tissue between the staple cartridge assembly and the anvil assembly for stapling. The surgical stapling instrument further includes an end component disposed at a front end of the anvil assembly, the end component being capable of extending into tissue and performing a separation operation on the tissue. In most surgical stapling instruments, the end component is integrally formed with the anvil assembly and is not detachable, and therefore the entire surgical stapling instrument must be replaced when the end component needs to be changed, which is extremely inconvenient. In a small number of surgical stapling instruments, the end component is detachably connected to the anvil assembly. However, the attachment and detachment of the end component require the use of auxiliary tools, which are time-consuming, labor-intensive, and relatively inconvenient to operate. In view of the foregoing, it is necessary to provide a surgical stapling instrument and an attachment and detachment method thereof to address the above-described issues.

### SUMMARY

The present disclosure aims to provide a surgical stapling instrument and an attachment and detachment method thereof that solve the technical problem in the prior art that the attachment and detachment of the end component require the use of auxiliary tools, which is time-consuming, labor-intensive, and relatively inconvenient to operate.

To achieve the objective, the present disclosure adopts the following technical solutions:

The present disclosure provides a surgical stapling instrument including a fixed anvil assembly; a cartridge assembly rotatably hinged to the anvil assembly; an end component detachably mounted on the anvil assembly and including a curved portion bent toward the cartridge assembly; wherein the end component includes a lumen opened toward a proximal end, and the anvil assembly includes a distal portion configured to be insertable into the lumen; the end component includes an elastic member extending outward from the lumen, and the distal portion is configured to be engageable with the elastic member; when the distal portion presses the elastic member to deform, the distal portion is configured to be partially inserted into or withdrawn from the lumen; when the distal portion is fully inserted into the lumen, the distal portion is configured to engage with the restored elastic member; when the distal portion is fully withdrawn from the lumen, the distal portion is configured to disengage from the restored elastic member.

The present disclosure also provides an attachment and detachment method of the surgical stapling instrument that includes: causing the distal portion to press the elastic member to deform, and to be partially inserted into the lumen; causing the distal portion to be fully inserted into the lumen and to engage with the restored elastic member; causing the distal portion to press the elastic member to deform, and to be partially withdrawn from the lumen; causing the distal portion to be fully withdrawn from the lumen and to disengage from the restored elastic member.

A surgical stapling instrument according to the present disclosure includes an end component provided with a lumen open toward a proximal end, and an anvil assembly provided with a distal portion configured to be inserted into the lumen. The end component further includes an elastic member extending outward from the lumen. When the distal portion presses the elastic member to deform, the distal portion is configured to be inserted into or withdrawn from the lumen. When the distal portion is fully inserted into the lumen, the distal portion engages with the restored elastic member. When the distal portion is fully withdrawn from the lumen, the distal portion disengages from the restored elastic member. The attachment and detachment of the end component of the surgical stapling instrument can be performed without auxiliary tools, making the operation more convenient.

An attachment and detachment method of a surgical stapling instrument according to the present disclosure includes the following steps: causing the distal portion to press the elastic member to deform, and to be partially inserted into the lumen; causing the distal portion to be fully inserted into the lumen and to engage with the restored elastic member; causing the distal portion to press the elastic member to deform, and to be partially withdrawn from the lumen; causing the distal portion to be fully withdrawn from the lumen and to disengage from the restored elastic member. The attachment and detachment of the end component of the surgical stapling instrument are relatively simple and do not require the use of auxiliary tools.

### BRIEF DESCRIPTION OF THE DRAWINGS

FIG. 1 is a schematic view of a surgical stapling instrument provided by the present disclosure.
FIG. 2 is a first schematic view of an actuator and an end component provided by the present disclosure.
FIG. 3 is a second schematic view of an actuator and an end component provided by the present disclosure.
FIG. 4 is a schematic view of the end component provided by the present disclosure.
FIG. 5 is a cross-sectional view of the end component provided by the present disclosure.
FIG. 6 is a schematic view of the structure of the end component and the distal portion to be attached provided by the present disclosure.
FIG. 7 is a first schematic view of the end component mounted on the distal portion provided by the present disclosure.
FIG. 8 is a second schematic view of the end component mounted on the distal portion provided by the present disclosure.
FIG. 9 is a third schematic view of the end component mounted on the distal portion provided by the present disclosure.
FIG. 10 is a first schematic view of the end component being detached from the distal portion provided by the present disclosure.

### Reference signs description:

10, surgical stapling instrument; 11, controller;
20, instrument shaft;
30, actuator; 31, cartridge assembly; 311, first tissue-contacting surface; 312, first longitudinal slot; 313, staple hole; 314, guide surface; 32, anvil assembly; 321, second tissue-contacting surface; 322, second longitudinal slot; 323, staple groove; 324, distal portion; 325, channel; 326, ramp surface; 33, pivot shaft; 34, driving assembly;
40, end component; 41, lumen; 411, first inner surface; 412, second inner surface; 42, curved portion; 43, elastic member; 431, inner side surface; 432, outer side surface; 433, first bending portion; 434, second bending portion; 44, groove.

### DETAILED DESCRIPTION

The following is a detailed description of the present disclosure in conjunction with the accompanying drawings and embodiments. It should be understood that, the specific embodiments described herein are merely for the purpose of explaining the present disclosure and are not intended to limit the present disclosure. In addition, it should be noted that, the drawings only illustrate parts related to the present disclosure and not the entire structure for clarity.

In the description of the present disclosure, unless otherwise explicitly specified or limited, the terms "connected to", "connected", and "fixed" should be broadly understood, for example, they can be understood as fixedly connected, detachably connected, or integrally formed, can be understood as mechanically or electrically connected, can be directly connected or indirectly connected through intervening media, or can be understood as connected through the use of two elements or the interaction of two elements. The specific meaning of the above terms in the present disclosure can be understood in detail by those skilled in the art.

In the present disclosure, unless otherwise expressly stated or limited, a first feature being "above" or "below" a second feature may include the first and second features being in direct contact, or that the first and second features being not in direct contact but being in contact through additional features between them. Further, a first feature being "on", "above" and "over" a second feature may include the first feature being directly above and diagonally above the second feature, or simply indicate that the first feature has a higher level than the second feature. The first feature being "under", "below" and "beneath" a second feature includes the first feature being directly below and diagonally below the second feature, or simply indicate that the first feature has a lower level than the second feature.

In the description of the present embodiment, the terms such as "upper", "lower", "right", which indicate orientations or positional relationships, are based on the orientations or positional relationships shown in the accompanying drawings, these terms are used merely for the convenience of description and to simplify the operation, and do not indicate or imply that the device or element must have a specific orientation, be constructed and operated in a specific orientation, and therefore should not be construed as limiting the present disclosure. Furthermore, the terms such as "first" and "second" are used merely for the purpose of distinction in description and do not have any special meaning.

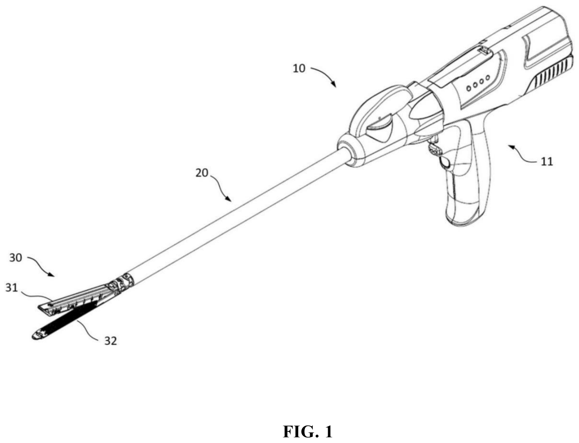

As shown in FIG. 1, the present embodiment provides a surgical stapling instrument 10 including a controller 11, an instrument shaft 20 extending distally from the controller 11, and an actuator 30 located at a distal end of the instrument shaft 20.

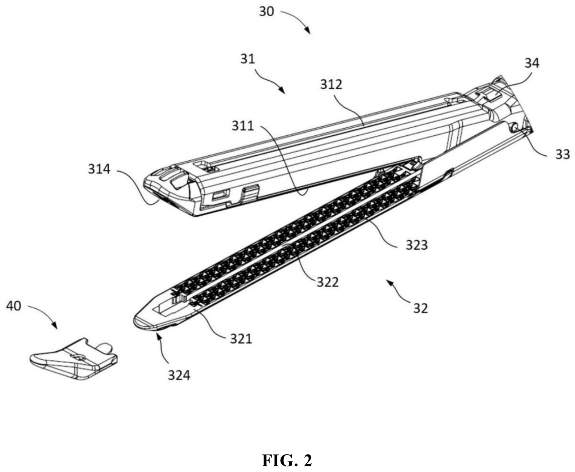

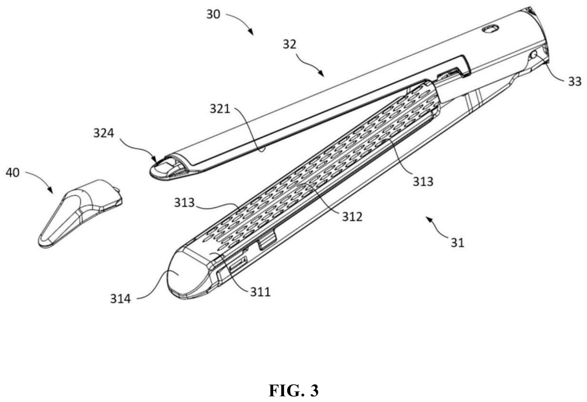

As shown in FIG. 2 and FIG. 3, the actuator 30 includes a pair of opposing jaws, which are configured as an anvil assembly 32 and a cartridge assembly 31. The proximal end of the cartridge assembly 31 is pivotably connected to the proximal end of the anvil assembly 32. The surfaces of the cartridge assembly 31 and the anvil assembly 32 that are opposite to each other are defined as their respective tissue-contacting surfaces. The controller 11 can control the actuator 30 to switch between an open state and a closed state. When the actuator 30 is in the open state, the first tissue-contacting surface 311 of the cartridge assembly 31 and the second tissue-contacting surface 321 of the anvil assembly 32 form an angle, and the most distal positions of the tissue-contacting surfaces are spaced away from each other. When the actuator 30 is in the closed state, the tissue-contacting surface of the cartridge assembly 31 and the tissue-contacting surface of the anvil assembly 32 are substantially parallel, and the most distal positions of the tissue-contacting surfaces are close to each other.

Referring to FIG. 2 and FIG. 3, the cartridge assembly 31 includes a first longitudinal slot 312. On two sides of the first longitudinal slot 312, three staggered rows of staple holes 313 are respectively provided on the first tissue-contacting surface 311. The staple holes 313 are provided with staples. The anvil assembly 32 includes a second longitudinal slot 322. On two sides of the second longitudinal slot 322, staple grooves 323 corresponding to the staple holes 313 are respectively provided on the second tissue-contacting surface 321. During operation, the user operates the controller 11 to close the jaws for clamping the target tissue. Then, by further operating the controller 11, multiple rows of staples in the cartridge assembly 31 are pushed out and formed in the staple grooves 323 of the anvil assembly 32, thereby stapling the tissue. In some stapling instruments, a cutting knife is further installed to cut the stapled tissue between multiple rows of staple lines.

It should be noted that a controller 11 may be driven by a motor or may be manually driven, and can control the actuator 30 to clamp and staple tissue. Similar technologies are publicly known and disclosed in various inventions, and therefore are not described in detail herein.

In the prior art, most surgical stapling instruments further include an end component. The end component is integrally formed with the anvil assembly and is not detachable, and therefore the entire surgical stapling instrument must be replaced when the end component needs to be changed, which is extremely inconvenient. In a small number of surgical stapling instruments, the end component is detachably connected to the anvil assembly. However, the attachment and detachment of the end component require the use of auxiliary tools, which are time-consuming, labor-intensive, and relatively inconvenient to operate.

As shown in FIG. 2 and FIG. 3, the surgical stapling instrument 10 further includes an end component 40, and the end component 40 may separate target tissue from specific tissue before the surgical stapling instrument 10 clamps the tissue. In this embodiment, the end component 40 is detachably mounted on the anvil assembly 32.

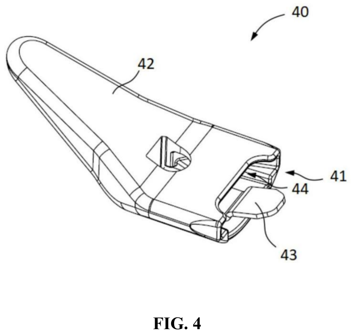

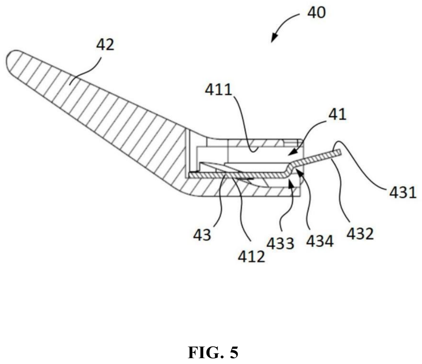

As shown in FIG. 4 and FIG. 5, the end component 40 has a lumen 41 that is open toward the proximal end. The anvil assembly 32 has a distal portion 324 configured to be inserted into the lumen 41. The end component 40 includes an elastic member 43 extending outward from the lumen 41, and the distal portion 324 is configured to engage with the elastic member 43. When the distal portion 324 presses the elastic member 43 to deform, the distal portion 324 is partially inserted into or withdrawn from the lumen 41. When the distal portion 324 is fully inserted into the lumen 41, the distal portion 324 engages with the restored elastic member 43. When the distal portion 324 is fully withdrawn from the lumen 41, the distal portion 324 disengages from the restored elastic member 43.

The end component 40 of the surgical stapling instrument 10 is detachably mounted on the distal portion 324 of the anvil assembly 32, reducing the burden on both the physician and the patient. No auxiliary tools are required during the attachment and detachment process, which makes the operation more convenient, easy to learn, and results in a low learning cost for the user.

Optionally, the shape of the lumen 41 matches the shape of the distal portion 324, and the distal portion 324 can be fitted into the lumen 41, so that the end component 40 does not wobble after being mounted on the distal portion 324, ensuring stable installation of the end component 40.

Optionally, the lumen 41 includes a first inner surface 411 located closer to the cartridge assembly 31, and a gap is provided between the elastic member 43 and the first inner surface 411 to allow insertion or withdrawal of the distal portion 324. The gap is capable of accommodating the distal portion 324.

Further, the first inner surface 411 can contact the surface of the distal portion 324 that is closer to the cartridge assembly 31, so that the end component 40 does not wobble after being mounted on the distal portion 324, ensuring stable installation of the end component 40.

Optionally, the lumen 41 includes a second inner surface 412 located away from the cartridge assembly 31, and a distance between the first inner surface 411 and the second inner surface 412 at the proximal end is greater than that at the distal end, so that the proximal end of the elastic member 43 has space for deformation.

Optionally, the elastic member 43 is deformable between the first inner surface 411 and the second inner surface 412 to vary the gap between the elastic member 43 and either the first inner surface 411 or the second inner surface 412, thereby enabling attachment and detachment of the end component 40 and the distal portion 324.

Optionally, the first inner surface 411 includes a groove 44 recessed from the proximal end toward the distal end. The provision of the groove 44 can provide an avoidance function for the distal portion 324 during installation of the end component 40 onto the distal portion 324, thereby preventing installation failure caused by interference between the end component 40 and the distal portion 324.

As shown in FIG. 5, the elastic member 43 includes a first bending portion 433 and a second bending portion 434 arranged from the distal end toward the proximal end. The first bending portion 433 bends toward the cartridge assembly 31, and the second bending portion 434 bends away from the cartridge assembly 31. The first bending portion 433 and the second bending portion 434 can interact with the distal portion 324 such that the elastic member 43 engages with the distal portion 324 when the end component 40 is installed onto the distal portion 324. When the end component 40 is detached from the distal portion 324, the interaction between the first bending portion 433, the second bending portion 434, and the distal portion 324 enables deformation of the elastic member 43, thereby allowing the end component 40 to disengage from the distal portion 324. Optionally, the proximal portion of the elastic member 43 extends outward from the lumen 41 and inclines toward the cartridge assembly 31. This allows the operator to distinguish the front and back orientation of the end component 40 by observing the proximal portion of the elastic member 43 extending out of the lumen 41, thereby preventing incorrect installation of the end component 40.

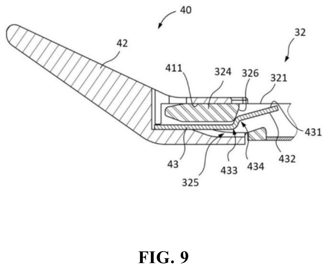

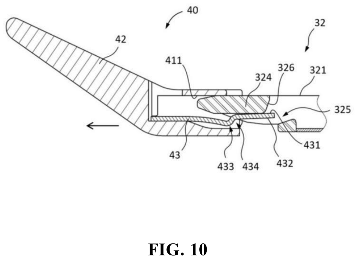

As shown in FIG. 9 and FIG. 10, the distal portion 324 includes a ramp surface 326 facing the proximal end, and the first bending portion 433 and the second bending portion 434 can interact with the ramp surface 326. Through the interaction between the first bending portion 433, the second bending portion 434, and the ramp surface 326, the ramp surface 326 prevents the end component 40 from disengaging from the distal portion 324.

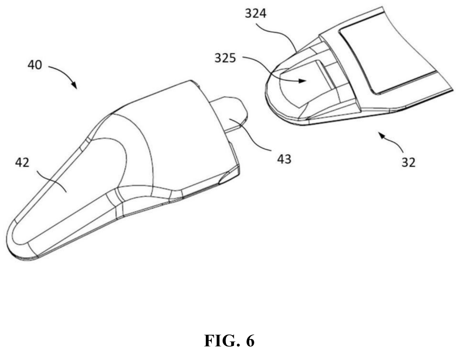

Optionally, as shown in FIG. 6, the anvil assembly 32 includes a channel 325 adjacent to the distal portion 324, and the channel 325 is configured to accommodate the proximal portion of the elastic member 43 so as to prevent the distal portion 324 from failing to extend into the end component 40.

The end component 40 in this embodiment is detachably mounted on the anvil assembly 32. According to user needs, the end component 40 may optionally be attached to the distal end of the anvil assembly 32 or detached from the distal end of the anvil assembly 32. The proximal end of the cartridge assembly 31 and the proximal end of the anvil assembly 32 are connected by a pivot shaft 33, and the two can switch between an open state and a closed state under the action of the driving assembly 34. In this embodiment, the anvil assembly 32 is fixed, and the cartridge assembly 31 is rotatably hinged to the anvil assembly 32. Specifically, the proximal end of the cartridge assembly 31 and the proximal end of the anvil assembly 32 are connected by the pivot shaft 33, and under the action of the driving assembly 34, the cartridge assembly 31 moves relative to the anvil assembly 32 between an open position and a closed position. The distal end of the cartridge assembly 31 is configured with a guide surface 314. The guide surface 314 is offset toward the solid portion of the cartridge assembly 31 and intersects with the most distal position of the first tissue-contacting surface 311.

When the actuator 30 is opened, the first tissue-contacting surface 311 and the second tissue-contacting surface 321 form an angle, and the most distal positions of the two are away from each other. When the actuator 30 is closed, the first tissue-contacting surface 311 and the second tissue-contacting surface 321 are substantially parallel, and the most distal positions of the two are close to each other. The cartridge assembly 31 includes a first longitudinal slot 312. On both sides of the first longitudinal slot 312, three staggered rows of staple holes 313 are respectively provided on the first tissue-contacting surface 311. The staple holes 313 are provided with staples. The anvil assembly 32 includes a second longitudinal slot 322. On both sides of the second longitudinal slot 322, staple grooves 323 corresponding to the staple holes 313 are respectively provided on the second tissue-contacting surface 321. During operation, the driving assembly 34 passes through the first longitudinal slot 312 and the second longitudinal slot 322, pushes out the staples from the cartridge assembly 31, and causes the staples to be formed within the staple grooves 323 of the anvil assembly 32.

Specifically, the end component 40 has a lumen 41 that is open toward the proximal end. The anvil assembly 32 has a distal portion 324 that can be inserted into the lumen 41. The distal portion 324 can extend into the lumen 41, thereby allowing the end component 40 to be mounted on the distal portion 324. In this embodiment, the end component 40 includes an elastic member 43 extending outward from the lumen 41, and the distal portion 324 can engage with the elastic member 43. In this embodiment, the elastic member 43 is made of a metal sheet, with its distal end fixed inside the lumen 41 and its proximal end extending outside the lumen 41. The proximal end of the elastic member 43 is designed with a chamfer to prevent the user from suffering accidental injury during operation. The elastic member 43 has an inner surface 431 and an outer surface 432. The inner surface 431 faces the side of the tissue-contacting surface closer to the anvil assembly 32, and the outer surface 432 faces the side of the tissue-contacting surface farther from the anvil assembly 32.

When the distal portion 324 presses the elastic member 43 to deform, the distal portion 324 partially inserts into or withdraws from the lumen 41. When the distal portion 324 is fully inserted into the lumen 41, the distal portion 324 engages with the restored elastic member 43. When the distal portion 324 is fully withdrawn from the lumen 41, the distal portion 324 disengages from the restored elastic member 43. The attachment and detachment of the end component 40 of the surgical stapling instrument 10 does not require auxiliary tools, making the operation more convenient.

As shown in FIG. 4, the end component 40 includes a mounting portion located at the proximal end and a curved portion 42 located at the distal end. The mounting portion and the curved portion 42 are arranged at an angle, and the curved portion 42 bends toward the cartridge assembly 31. The cross-section of the curved portion 42 gradually decreases from the proximal end to the distal end to enhance guidance and facilitate the anvil assembly 32 passing through the target tissue. Its most distal end is configured as an arc shape to avoid causing injury to the patient. During detachment of the end component 40, a horizontal force is applied to the curved portion 42 away from the distal portion 324 to disengage the end component 40 from the distal portion 324. The provision of the curved portion 42 facilitates the user to hold the end component 40 for attachment and detachment.

Furthermore, the shape of the lumen 41 matches that of the distal portion 324. The distal portion 324 is engaged with the lumen 41, thereby enabling the stable installation of the end component 40 on the distal portion 324. Specifically, the distal portion 324 is configured as a wedge-shaped structure, i.e., the distal portion 324 gradually tapers in the width direction to facilitate passage through the target tissue. The lumen 41 is arranged at the proximal end of the end component 40, with its width smaller than the width of the outer surface of the end component 40. Considering that the width of the end component 40 is comparable to that of the anvil assembly 32, the gradually tapered distal portion 324 can be inserted into the lumen 41. The lumen 41 is fitted to the distal portion 324 to limit relative movement between the end component 40 and the anvil assembly 32.

Furthermore, the lumen 41 has a first inner surface 411 near the cartridge assembly 31 and a second inner surface 412 away from the cartridge assembly 31, with the elastic member 43 disposed between the first inner surface 411 and the second inner surface 412. A gap is provided between the elastic member 43 and the first inner surface 411 to allow the distal portion 324 to be inserted or withdrawn, and the gap can accommodate the distal portion 324. After the distal portion 324 is inserted into the gap, the first inner surface 411 can contact the surface of the distal portion 324 adjacent to the cartridge assembly 31, so that the end component 40 does not wobble when mounted on the distal portion 324, ensuring stable installation. The distance between the first inner surface 411 and the second inner surface 412 at the proximal end is greater than that at the distal end, allowing the elastic member 43 to deform between the first inner surface 411 and the second inner surface 412, thereby changing the gap between the elastic member 43 and the first inner surface 411 or the second inner surface 412 and thus achieving the attachment and detachment of the end component 40 with the distal portion 324.

In this embodiment, the first inner surface 411 is provided with a groove 44 recessed from the proximal end toward the distal end, which provides clearance for the distal portion 324 during installation of the end component 40 on the distal portion 324, preventing interference between the end component 40 and the distal portion 324 that would hinder installation.

As shown in FIG. 5, in this embodiment, the elastic member 43 includes a first bending portion 433 and a second bending portion 434 arranged from the distal end toward the proximal end. The first bending portion 433 bends toward the cartridge assembly 31, i.e., the first bending portion 433 bends toward the inner surface 431, while the second bending portion 434 bends away from the cartridge assembly 31, i.e., the second bending portion 434 bends toward the outer surface 432. The first bending portion 433 and the second bending portion 434 can interact with the distal portion 324, so that when the end component 40 is mounted on the distal portion 324, the elastic member 43 engages with the distal portion 324. When the end component 40 is disengaged from the distal portion 324, the interaction between the first bending portion 433, the second bending portion 434, and the distal portion 324 causes the elastic member 43 to deform, allowing the detachment of the end component 40 from the distal portion 324.

Optionally, the proximal portion of the elastic member 43 (the part of the elastic member 43 near the second bending portion 434) extends outward from the lumen 41 and is inclined toward the cartridge assembly 31, allowing the operator to distinguish the correct orientation of the end component 40 and avoid incorrect installation. As shown in FIG. 6, the anvil assembly 32 has a channel 325 adjacent to the distal portion 324, and the channel 325 can accommodate the proximal portion of the elastic member 43, preventing the distal portion 324 from failing to be inserted into the end component 40.

To prevent the end component 40 from disengaging from the distal portion 324 after being mounted, the distal portion 324 has a ramp surface 326 facing toward the proximal end (as shown in FIG. 9 and FIG. 10). The first bending portion 433 and the second bending portion 434 can interact with the ramp surface 326. The ramp surface 326 can hinder the detachment of the end component 40, so that the ramp surface 326 can prevent the end component 40 from disengaging from the distal portion 324.

When the distal portion 324 presses the elastic member 43 causing it to deform, the distal portion 324 partially inserts into or withdraws from the lumen 41. When the distal portion 324 is fully inserted into the lumen 41, the ramp surface 326 of the distal portion 324 engages with the restored elastic member 43. When the distal portion 324 fully exits from the lumen 41, the distal portion 324 disengages from the restored elastic member 43.

In the present disclosure, the description of orientations is generally made with reference to the operator or the patient of the medical instrument. The position closer to the operator of the medical instrument is referred to as the "proximal" end, and the position closer to the patient is referred to as the "distal" end.

The present disclosure further provides an attachment and detachment method for the surgical stapling instrument, namely, a method for attaching and detaching the end component 40. The attachment and detachment method of the surgical stapling instrument includes the following steps:
Causing the distal portion 324 to press the elastic member 43 to deform, and to be partially inserted into the lumen 41;
Causing the distal portion 324 to be fully inserted into the lumen 41 and to engage with the restored elastic member 43;
Causing the distal portion 324 to press the elastic member 43 to deform, and to be partially withdrawn from the lumen 41;
Causing the distal portion 324 to be fully withdrawn from the lumen 41 and to disengage from the restored elastic member 43.

Through the above attachment and detachment method of the surgical stapling instrument 10, the end component 40 can be attached and detached in a relatively simple manner without the use of auxiliary tools.

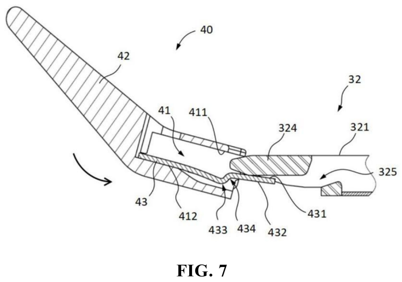

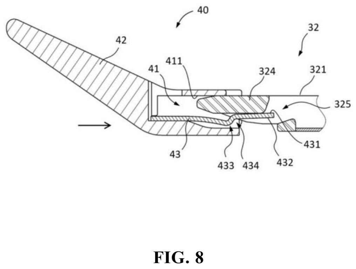

Specifically, as shown in FIG. 7, when installing the end component 40, the end component 40 forms a slight inclination angle relative to the distal portion 324, so that a portion of the distal portion 324 is inserted into the lumen 41 through the opening of the lumen 41. During this process, the distal end of the distal portion 324 presses against the elastic member 43, causing the elastic member 43 to bend and deform in a direction away from the first inner surface 411. As shown in FIG. 8, when the end component 40 slides toward the proximal end by a certain distance, the first inner surface 411 of the lumen 41 comes into contact with the tissue-contacting surface of the distal portion 324. At this moment, the elastic member 43 reaches its maximum deformation, and its proximal end continues sliding toward the proximal direction along the channel 325, enabling the distal portion 324 to further extend into the lumen 41 in parallel with the first inner surface 411. As shown in FIG. 9, when the distal portion 324 is fully inserted into the lumen 41, the elastic member 43 recovers from deformation, and the ramp surface 326 of the distal portion 324 engages with the first bending portion 433 and the second bending portion 434 of the recovered elastic member 43. The installation is thereby completed, and at this moment the distal portion 324 is in contact with the first inner surface 411.

During use, the end component 40 interacts with tissue with a relatively small force, since an excessive force may damage the tissue. The engagement force generated between the elastic member 43 and the ramp surface 326 is sufficient to prevent the end component 40 from separating from the anvil assembly 32.

As shown in FIG. 10, when detaching the end component 40, the user holds the end component 40 and applies a horizontal force thereto in a direction away from the distal portion 324. The ramp surface 326 of the distal portion 324 presses against the elastic member 43, causing the elastic member 43 to bend and deform in a direction away from the first inner surface 411, thereby disengaging the elastic member 43 from the ramp surface 326 and allowing the distal portion 324 to partially withdraw from the lumen 41. By continuously applying the horizontal force, the end component 40 completely separates from the distal portion 324, and the restored elastic member 43 disengages from the distal portion 324, thereby completing the detachment of the end component 40 from the anvil assembly 32.

Through the above-described attachment and detachment method, the end component 40 of the surgical stapling instrument 10 can be attached and detached without the use of auxiliary tools, thereby providing a convenient attachment and detachment process.

It is apparent that the embodiments above of the present disclosure are merely examples provided for clearly illustrating the present disclosure, and are not intended to limit the present disclosure. For those of ordinary skill in the art, other variations or modifications in different forms may be made based on the above description. It is neither necessary nor possible to exhaustively enumerate all embodiments herein. Any modifications, equivalent substitutions, improvements, and the like made within the spirit and principles of the present disclosure shall be included within the scope of protection of the claims of the present disclosure.

## Claims

1. A surgical stapling instrument comprising:
a fixed anvil assembly (32);
a cartridge assembly (31) rotatably hinged to the anvil assembly (32); and
an end component (40) detachably mounted on the anvil assembly (32) and comprising a curved portion (42) bent toward the cartridge assembly (31);
wherein the end component (40) comprises a lumen (41) opened toward a proximal end, and the anvil assembly (32) comprises a distal portion (324) configured to be insertable into the lumen (41);
the distal portion (324) comprises a ramp surface (326) facing the proximal end; the end component (40) comprises an elastic member (43) extending outward from the lumen (41), and the ramp surface (326) is configured to be engageable with the elastic member (43); the lumen (41) comprises a first inner surface (411) located near the cartridge assembly (31); a gap is provided between the elastic member (43) and the first inner surface (411), which is configured to allow the distal portion (324) to be inserted or withdrawn; the lumen (41) comprises a second inner surface (412) located away from the cartridge assembly (31), and the elastic member (43) is configured to be deformable between the first inner surface (411) and the second inner surface (412), which is configured to change the gap between the elastic member (43) and either the first inner surface (411) or the second inner surface (412);
when the distal portion (324) presses the elastic member (43) to deform, the distal portion (324) is configured to be partially inserted into or withdrawn from the lumen (41); when the distal portion (324) is fully inserted into the lumen (41), the distal portion (324) is configured to engage with the restored elastic member (43);
when the distal portion (324) is fully withdrawn from the lumen (41), the distal portion (324) is configured to disengage from the restored elastic member (43).

2. The surgical stapling instrument according to claim 1, wherein a shape of the lumen (41) is adapted to a shape of the distal portion (324), and the distal portion (324) is configured to be fitted with the lumen (41).

3. The surgical stapling instrument according to claim 1, wherein a proximal portion of the elastic member (43) extends outward from the lumen (41) and is inclined toward the cartridge assembly (31).

4. The surgical stapling instrument according to claim 1, wherein the first inner surface (411) is configured to contact a surface of the distal portion (324) that is located near the cartridge assembly (31).

5. The surgical stapling instrument according to claim 1, wherein the first inner surface (411) comprises a groove (44) recessed from a proximal end toward a distal end.

6. The surgical stapling instrument according to claim 1, wherein a distance between the first inner surface (411) and the second inner surface (412) at a proximal end is greater than a distance therebetween at a distal end.

7. The surgical stapling instrument according to claim 1, wherein the elastic member (43) comprises a first bending portion (433) and a second bending portion (434) arranged from a distal end toward a proximal end, wherein the first bending portion (433) is bent toward the cartridge assembly (31), the second bending portion (434) is bent away from the cartridge assembly (31), and the first bending portion (433) and the second bending portion (434) are configured to interact with the distal portion (324).

8. The surgical stapling instrument according to claim 7, wherein the first bending portion (433) and the second bending portion (434) are configured to interact with the ramp surface (326).

9. The surgical stapling instrument according to claim 1, wherein the anvil assembly (32) comprises a channel (325) adjacent to the distal portion (324), and the channel (325) is configured to accommodate a proximal portion of the elastic member (43).

10. An attachment and detachment method of a surgical stapling instrument, wherein the surgical stapling instrument comprises a fixed anvil assembly (32); a cartridge assembly (31) rotatably hinged to the anvil assembly (32); and an end component (40) detachably mounted on the anvil assembly (32) and comprising a curved portion (42) bent toward the cartridge assembly (31); wherein the end component (40) comprises a lumen (41) opened toward a proximal end, and the anvil assembly (32) comprises a distal portion (324) configured to be insertable into the lumen (41); the distal portion (324) comprises a ramp surface (326) facing the proximal end; the end component (40) comprises an elastic member (43) extending outward from the lumen (41), and the ramp surface (326) is configured to be engageable with the elastic member (43); the lumen (41) comprises a first inner surface (411) located near the cartridge assembly (31); a gap is provided between the elastic member (43) and the first inner surface (411), which is configured to allow the distal portion (324) to be inserted or withdrawn; the lumen (41) comprises a second inner surface (412) located away from the cartridge assembly (31), and the elastic member (43) is configured to be deformable between the first inner surface (411) and the second inner surface (412), which is configured to change the gap between the elastic member (43) and either the first inner surface (411) or the second inner surface (412);
wherein the attachment and detachment method comprises:
causing the distal portion (324) to press the elastic member (43) to deform, and to be partially inserted into the lumen (41);
causing the distal portion (324) to be fully inserted into the lumen (41) and to engage with the restored elastic member (43);
causing the distal portion (324) to press the elastic member (43) to deform, and to be partially withdrawn from the lumen (41);
causing the distal portion (324) to be fully withdrawn from the lumen (41) and to disengage from the restored elastic member (43).
